# FASCICULE DE BREVET EUROPEEN

(11) **EP 0 922 071 B1**
(45) Date de publication et mention de la délivrance du brevet: **14.11.2001**
(21) Numéro de dépôt: 97938958.2
(22) Date de dépôt: 29.08.1997
(51) Int. Cl.: C08K 5/17, C08B 37/08, C08B 37/06, C08B 37/10, C08L 5/08, C08L 33/06, A61K 9/20

(54) **COPOLYMERES RETICULES A BASE DE POLYMERES POLYCARBOXYLIQUES**
VERNETZTE COPOLYMERE AUF DER BASIS VON POLYCARBONSÄUREPOLYMEREN
POLYCARBOXYLIC BASED CROSS-LINKED COPOLYMERS

(30) Priorité: 30.08.1996 FR 9610601
(43) Date de publication de la demande: 16.06.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: EL MATNI, Nada, F-75116 Paris (FR); LABARRE, Denis, F-91140 Villebon sur Yvette (FR); FESSIM, Hatem, F-69009 Lyon (FR)
(74) Mandataire: Bourgouin, André
(86) Numéro de dépôt international: FR9701534
(87) Numéro de publication internationale: WO9808897

(56) Documents cités:
- EP-A- 0 334 167
- WO-A-89/02445
- WO-A-91/16881
- US-A- 4 026 851
- US-A- 5 017 229

## Description

L'invention concerne des copolymères réticulés à base de polymères polycarboxyliques non réticulés, les dits copolymères contenant au moins un polysaccharide polycarboxylique. L'invention concerne également un procédé de préparation de ces copolymères et leur utilisation notamment comme support dans les compositions pharmaceutiques.

Certains composés à structure polymérique contenant un polysaccharide polycarboxylique, éventuellement modifié, ont été décrits dans la littérature. Par exemple, la demande WO89/02445 décrit un gel à base d'acide hyaluronique ; mais ce gel ne comprend, dans sa structure, que de l'acide hyaluronique et aucun autre polymère polycarboxylique. Par ailleurs, aucun agent de réticulation est utilisé par la préparation de ce gel. Le composé ainsi obtenu est principalement utilisé en chirurgie. La demande WO91/16881 décrit, entre autre, la combinaison d'un principe actif avec une matrice constitué d'un polymère modifié, c'est à dire sur lequel sont greffés des saccharides. Ce polymère modifié peut être un polymère naturel tel que la chondroïtine sulfate. Mais cette matrice ne contient qu'un seul type de polymère.

Les copolymères selon l'invention à base de polymères polycarboxyliques, contiennent au moins un polysaccharide polycarboxylique et au moins un autre polymère polycarboxylique qui n'est pas un polysaccharide. L'association d'un polysaccharide avec un autre type de polymère polycarboxylique, permet de moduler les propriétés des polysaccharides telles que l'hydrophilie. On peut ainsi obtenir des copolymères avec des propriétés de dégradation appropriées en fonction de leurs applications. Par ailleurs, les copolymères selon l'invention, sont avantageusement préparés en milieu aqueux. Ceci est un réel avantage car il est presque impossible d'éliminer totalement les solvants dans une structure polymérique : l'existence de traces de solvants aqueux résiduels est en général plus facilement acceptable et acceptée que des traces de solvants organiques résiduels tels que le diméthylsulfoxyde ou la diméthylformamide.

L'invention a pour objet des copolymères réticulés à base de polymères polycarboxyliques non réticulés et d'un agent de réticulation comprenant au moins deux fonctions amine, les dits copolymères comprenant au moins un polysaccharide polycarboxylique et au moins un autre polymère polycarboxylique non réticulé qui n'est pas un polysaccharide polycarboxylique.

Les polysaccharides polycarboxyliques non réticulés peuvent être choisis, par exemple, parmi les glycosaminoglycanes, l'acide pectinique, l'acide alginique, les dérivés carboxyliques du dextrane tels que les carboxyméthyldextranes, ou les dérivés carboxyliques de la cellulose tels que les carboxyméthylcelluloses. Parmi les glycosaminoglycanes, on peut citer l'acide hyaluronique, la chondroïtine sulfate, l'héparine, le sulfate de dermatane, le sulfate d'héparane, le sulfate de kératane ou un mélange de ces derniers. Parmi les polymères polycarboxyliques qui ne sont pas des polysaccharides, on peut citer le poly(acide glutamique), le poly(acide aspartique), le poly(acide maléique), le poly(acide malique) ou le poly(acide fumarique), les polymères acryliques polycarboxyliques tels que le poly(acide acrylique), le poly(acide méthacrylique) ou les copolymères de ces derniers tels que les Eudragits® L et S. L'expression polymères polycarboxyliques comprend les polymères tels que définis ci-dessus mais également les dérivés partiellement ou totalement substitués de ces polymères comme, par exemple, leurs esters, leurs amides ou leurs sels, les copolymères contenant les unités présentes dans ces polymères polycarboxyliques ou dans leurs dérivés tels que définies ci-dessus, mais aussi le mélange de ces polymères et/ou de leurs dérivés et/ou de leurs copolymères tels que définis ci-dessus.

L'invention a plus particulièrement pour objet des copolymères réticulés tels que définis ci-dessus, caractérisés en ce que le polysaccharide est choisi parmi l'acide pectinique ou alginique, les glycosaminoglycanes, et de préférence l'acide hyaluronique, la chondroïtine sulfate, l'héparine, le sulfate de dermatane, le sulfate d'héparane, le sulfate de kératane ou un mélange de ces derniers.

L'invention a plus particulièrement pour objet des copolymères réticulés tels que définis ci-dessus, caractérisés en ce que le polymère polycarboxylique non réticulé qui n'est pas un polysaccharide polycarboxylique est choisi parmi les polymères acryliques polycarboxyliques, le poly(acide glutamique), le poly(acide aspartique), le poly(acide maléique), le poly(acide malique) ou le poly(acide fumarique). De préférence, le polymère polycarboxylique non réticulé qui n'est pas un polysaccharide polycarboxylique, est un polymère acrylique polycarboxylique et plus particulièrement le poly(acide acrylique) ou le poly(acide méthacrylique).

Les polymères polycarboxyliques selon l'invention, sont liés entre eux par un agent de réticulation. Cet agent de réticulation comprend au moins deux fonctions amines qui sont susceptibles de réagir avec les fonctions carboxyliques libres des dits polymères carboxyliques non réticulés. Il peut être choisi, par exemple, parmi les protéines, les polyamines, les triamines, les diamines, les acides aminés naturels ou synthétiques, ou les dérivés des composés tels que définis ci-dessus comme, par exemple, leurs sels, leurs esters ou leurs amides. Parmi les acides aminés, on peut citer, par exemple, l'arginine, la lysine, l'histidine et l'ornithine. Parmi les diamines, on peut citer l'éthylènediamine, la butanediamine, l'hexanediamine, l'heptanediamine, l'octanediamine ou la dodécanediamine. Parmi les polyamines, on peut citer le chitosane, les poly(acide aminé) tels que la polylysine ou la polyornithine, ainsi que les copolymères de ces polyamines. L'agent de réticulation peut également être choisi parmi les composés tels que la spermine, la spermidine, la mélamine, la guanidine ou la diéthylènetriamine. De préférence, l'agent de réticulation utilisé est un acide aminé et avantageusement la lysine, l'ornithine ou l'histidine.

L'invention a plus particulièrement pour objet également des copolymères réticulés tels que définis ci-dessus, caractérisés en ce que le polysaccharide polycarboxylique est un polysaccharide polycarboxylique dégradable par'la flore microbienne du colon tel que la chondroïtine sulfate, l'acide hyaluronique, l'acide pectinique ou l'héparine.

L'invention a plus particulièrement pour objet des copolymères réticulés tels que définis ci-dessus, caractérisés en ce que le polysaccharide polycarboxylique est la chondroïtine sulfate et l'autre dit polymère polycarboxylique est choisi parmi le poly(acide acrylique) et le poly(acide méthacrylique), et l'agent de réticulation est la lysine ou l'histidine.

L'invention a également pour objet un procédé de préparation de copolymères réticulés tels que définis ci-dessus, ledit procédé caractérisé en ce que l'on fait réagir les dits polymères polycarboxyliques non réticulés constituant le copolymère réticulé, en présence d'un activateur et d'un agent de réticulation comprenant au moins deux fonctions amine, dans un milieu réactionnel approprié. De préférence, la préparation de copolymères réticulés tels que définis ci-dessus s'effectue en milieu aqueux. L'expression milieu aqueux signifie un milieu ne contenant que de l'eau ou de l'eau mélangée avec un ou plusieurs solvants miscibles à l'eau tel que, par exemple, l'acétone ou les alcools inférieurs tels que l'éthanol. De préférence le milieu aqueux ne comprend que de l'eau. La mise en oeuvre du procédé selon l'invention peut s'effectuer de différentes manières. En effet, le procédé peut consister à mélanger ensemble les polymères polycarboxyliques non réticulés et l'agent de réticulation, puis à rajouter l'activateur. Le procédé de réticulation selon l'invention peut consister également à mélanger ensemble les polymères polycarboxyliques non réticulés et l'activateur, puis à rajouter l'agent de réticulation. Le procédé peut consister également à réticuler un des polymères polycarboxyliques non réticulés constituant du copolymère, en mélangeant ce dit polymère avec l'agent de réticulation puis l'activateur ou bien avec l'activateur puis l'agent de réticulation, puis à rajouter dans le milieu réactionnel au moins un autre polymère polycarboxylique non réticulé, pour le réticuler avec le dit polymère présent dans le mélange réactionnel. Lors de la mise en oeuvre du procédé, les réactifs mis en présence peuvent être préalablement solubilisés dans le milieu réactionnel choisi. De préférence, les polymères polycarboxyliques non réticulés et l'agent de réticulation sont mélangés ensemble dans un milieu aqueux jusqu'à solubilisation puis l'activateur est rajouté. Le procédé est mis en oeuvre à une température comprise entre -30 et 100°C, de préférence entre 0 et 40 °C et de manière très préférentielle à température ambiante. La température de mise en oeuvre du procédé de réticulation est bien évidemment inférieure aux températures de dégradation ou de décomposition des réactifs mis en présence.

Les proportions relatives des réactifs que sont les polymères polycarboxyliques non réticulés, l'agent de réticulation et l'activateur, peuvent varier selon les caractéristiques des copolymères recherchés. Les proportions des polymères polycarboxyliques non réticulés sont définies par rapport aux quantités molaires des fonctions carboxyliques présentes par unité de base. Les polymères polycarboxyliques non réticulés peuvent varier dans un rapport molaire compris entre 0,01 et 100. Le rapport molaire de l'agent de réticulation par rapport aux fonctions carboxyliques totales peut varier entre 0,01 et 100. Le rapport molaire de l'activateur par rapport aux fonctions carboxyliques totales peut varier entre 0,01 et 100.

L'activateur peut être choisi parmi les agents de couplage classiquement utilisés en synthèse peptidique. Ainsi l'activateur peut être choisi, par exemple, parmi les carbodiimides, des dérivés des quinolines ou des anhydrides mixtes. Comme exemple de carbodiimides, on peut citer les hydrohalogénures tels que l'hydrochlorure de N-(3-diméthylaminopropyl)-N'-éthyl carbodiimide (EDC), le N-cyclohexyl-N'-(2-morpholinoéthyl) carbodiimide (CMC). Comme exemple des dérivés des quinolines, on peut citer la 2-éthoxy-N-éthoxycarbonyl-1,2-dihydroquinoline (EEDQ), la N-isobutoxycarbonyl-2-isobutoxy-1,2-dihydroquinoline (IIDQ), la N-isobutoxycarbonyl-2-méthoxy-1,2-dihydroquinoline (IMDQ), la N-isobutoxycarbonyl-2-éthoxy-1,2-dihydroquinoline (IEDQ). Comme exemple d'anhydrides mixtes, on peut 5 citer les chloroformates et plus particulièrement l'isobutylchloroformate (IBC). De préférence, l'activateur utilisé est l'hydrochlorure de N-(3-diméthylaminopropyl)-N'-éthyl carbodiimide.

Les copolymères réticulés selon l'invention peuvent être utilisés, par exemple, dans les domaines pharmaceutiques, cosmétiques, biomédicaux, vétérinaires, chimiques, agrochimiques ou agroalimentaires.

Plus particulièrement, l'invention a pour objet une composition pharmaceutique contenant au moins un principe actif et, à titre de support inerte ou d'excipient, au moins un copolymère réticulé selon l'invention. L'expression principe actif désigne toute substance ou mélange de substances ayant une activité thérapeutique.

Une telle composition peut être élaborée à partir de ces différents composants par toute technique classique connue de l'homme de l'art. Elle peut se présenter, par exemple, sous forme de comprimés matriciels, de comprimés enrobés par les copolymères de la présente invention, de comprimés multicouches, de pellets matriciels, de pellets ou des microparticules enrobés par les copolymères de la présente invention. Ces microparticules et pellets peuvent être contenus ou non dans des capsules. Elle peut se présenter également sous forme de microparticules ou de nanoparticules dont l'un au moins des constituants est un copolymère de la présente invention ou bien sous toute autre forme permettant une administration orale. Elle peut se présenter également sous toute autre forme adaptée au mode d'administration choisi ou approprié telle que des suppositoires ou des préparations pour application locale ou pour injection. La quantité du principe actif permettant une action pharmacologique efficace, en particulier thérapeutique, peut varier en fonction de la nature du principe actif, de l'âge et/ou de la maladie du patient à traiter.

La présente invention a également pour objet l'utilisation d'une composition pharmaceutique selon l'invention pour une libération contrôlée du ou des principes actifs qu'elle contient.

De telles compositions peuvent également posséder d'autres caractéristiques qui dépendent éventuellement des caractéristiques des polymères polycarboxyliques de départ telles que la bioadhésion. Ainsi, une composition pharmaceutique selon l'invention peut également être utilisée en tant que système pharmaceutique biadhésif. La présente invention a donc également pour objet l'utilisation d'une composition pharmaceutique selon l'invention en tant que système bioadhésif.

Des compositions telles définies ci-dessus dans lesquelles le polysaccharide polycarboxylique est dégradable par la flore du colon, peuvent également être utilisée en tant que système à libération spécifique au niveau du colon par action de la flore microbienne. Le concept de la libération spécifique au niveau du colon par action de la flore microbienne, est basé sur la propriété du colon de posséder une flore microbienne très abondante qui, de plus, a la potentialité de métaboliser des substances faiblement ou non dégradées par la partie haute du tube digestif. De telles compositions sont particulièrement adaptées pour véhiculer des principes actifs destinés au traitement des maladies du colon, ce qui permet d'augmenter leur efficacité et de diminuer leurs effets secondaires. Parmi ces principes actifs figurent les stéroïdes telles que la dexamethasone et l'hydrocortisone, les anti-inflammatoires non stéroïdiens tels que l'acide 5-aminosalicylique, les antinéoplasiques tels que le methotrexate, le tamoxifène, les antispasmodiques et les agents chimiothérapiques. De telles compositions sont particulièrement adaptées également pour véhiculer des principes actifs qui sont absorbés de façon plus efficace au niveau du colon tels que les stéroïdes ou la xanthine. Leur administration directe au niveau du colon permet d'augmenter leur efficacité. De telles compositions sont particulièrement adaptées également pour véhiculer des principes actifs qui sont dégradés dans les parties hautes du tube digestif. Parmi ces principes actifs, on peut citer les peptides et les protéines tels que les vaccins oraux, l'insuline, les peptides contraceptifs, les peptides activateurs du plasminogène, les peptides de croissance, LH/RH.

Les exemples suivants sont présentés pour illustrer les procédures ci-dessus et ne doivent en aucun cas être considérés comme une limite à la portée de l'invention.

### PARTIE EXPÉRIMENTALE

### EXEMPLE 1

1,33 g de sel sodique de chondroitine sulfate (A à 70%, C à 30 %) (CS), 0,29 g de sel sodique d'acide polyméthacrylique (PMA) et 3,35 g de monochlorhydrate de L-lysine sont mélangés ensemble dans 9 ml d'eau bidistillée jusqu'à obtention d'une solution limpide qui est par la suite dégazée. Puis on ajoute 4,59 g de chlorhydrate de N-éthyl-N'-(3-diméthylaminopropyl) carbodiimide (EDC). Le pH est maintenu entre 6 et 7 par ajout successif d'acide chlorhydrique 2,5 N. La réaction est effectuée à température ambiante pendant 6 heures. Puis le milieu réactionnel est transvasé dans un boudin de dialyse (Spectra/por, de seuil de coupure 12-14 KD) et dialysé 4 fois contre 4 litres d'eau à chaque fois. Le précipité ainsi obtenu est lavé à l'eau puis séché. On obtient le copolymère de chondroïtine sulfate et d'acide polyméthacrylique recherché de masse moyenne 1,53 ± 0,12 g. L'utilisation du soufre comme marqueur de la chondroïtine sulfate permet de définir, par une analyse élémentaire, le pourcentage en masse de chondroïtine sulfate dans le copolymère qui est égal à 59 ± 2 %.

### EXEMPLE 2

On travaille de la même manière que dans l'exemple 1, mais en utilisant 1,77 g de monochlorhydrate de L-lysine et 2,76 g d'EDC. La masse du copolymère obtenu est de 1,06 ± 0,15 g ; le pourcentage en masse de CS dans le précipité est de 55 ± 2.

### EXEMPLE 3

On travaille de la même manière que dans l'exemple 1, mais en utilisant 7,06 g de monochlorhydrate de L-lysine et 8,21 g d'EDC. La masse du copolymère obtenu est de 1,61 ± 0,12 g ; le pourcentage en masse de CS dans le précipité est de 61 ± 1.

### EXEMPLE 4

On travaille de la même manière que dans l'exemple 1, mais en utilisant 3 g d'histidine à la place de la L-Lysine. La masse du copolymère obtenu est de 1,94 ± 0,01 g ; le pourcentage en masse de CS dans le précipité est de 48 ± 3.

### EXEMPLE 5

On travaille de la même manière que dans l'exemple 1, mais en utilisant 0,43 g de PMA, 4,5 g de monochlorhydrate de L-lysine et 5,82 g d'EDC. La masse du copolymère obtenu est de 1,86 ± 0,05 g ; le pourcentage en masse de CS dans le précipité est de 58 ± 2.

### EXEMPLE 6

On travaille de la même manière que dans l'exemple 1, mais en utilisant 0,58 g de PMA, 5,45 g de monochlorhydrate de L-lysine et 7,05 g d'EDC. La masse du copolymère obtenu est de 2,07 ± 0,01 g ; le pourcentage en masse de CS dans le précipité est de 54 ± 2.

### EXEMPLE 7

Des essais de solubilisation du copolymère de l'exemple 1 sont effectués dans les solvants et mélanges de solvants suivants : eau à pH 3 et 7, acétonitrile, éthanol, tétrahydrofurane, dichlorométhane, diméthylsulfoxyde, diméthylacétamide, acétone, dioxane, triéthylamine, chloroforme, éther de pétrole, hexane, diméthylformamide, alcool benzylique, heptane, alcool isopropylique, propanediol-1,2, mélange eau / acétone (50%/50%), mélange eau /éthanol (50%/50%).
Le copolymère est insoluble dans tous ces solvants ce qui montre son caractère réticulé.

### Etude de la dégradation enzvmatique de copolymères

### 1- étude spectroscopique

Nous étudions ici la dégradation des copolymères de l'invention à base de chondroïtine sulfate, par les chondroïtinases, enzymes de la flore microbienne du colon.

Des suspensions de copolymères des exemples 1 à 6, dans un tampon (acétate/tris/albumine) de pH 7,3, sont préparées et agitées pour se stabiliser quelques heures. Les suspensions contiennent 67 mg de CS/ml de tampon. Une solution de chondroïtinases est rajoutée à raison de 3.10⁻³ UE (Unité Enzymatique) pour chaque mg de CS contenue dans la suspension. Le mélange est incubé à 37°C. A des temps déterminés, une suspension est centrifugée à 4°C puis filtrée. Ensuite, une étude de l'absorbance UV du surnageant est effectuée. Les disaccharides provenant de la dégradation de la CS ont un maximum d'absorption à 230-240 nm (Yamagata, T.et al., *J.Biol.Chem*., 243(7):1523-1535(1968) ; Salyers, A. et coll., *J.Bactériol*., 143(2):772-780)). Le témoin est une solution de CS non réticulée préparée dans les mêmes conditions opératoires que ci-dessus.

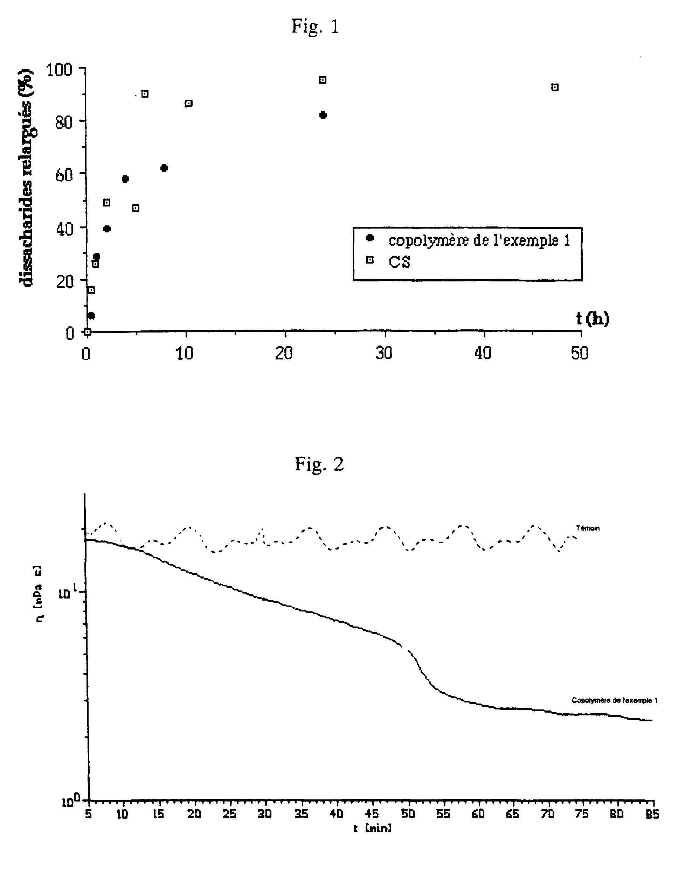

Les cinétiques d'apparition en solution des disaccharides provenant de la dégradation de la CS non réticulée et du copolymère obtenu dans l'exemple 1 sont présentées dans la figure 1 ci-après.

Ces résultats montrent que le copolymère de l'exemple 1 est dégradé par les enzymes. La comparaison de la dégradation du copolymère de l'exemple 1 avec celle du témoin, montre que le copolymère, bien que réticulé, est rapidement dégradé par les enzymes.

On procède de la même façon sur les copolymères des exemples 2 à 6 ; les résultats montrent que ces copolymères contenant la CS sont dégradés par les chondroïtinases.

### 2- étude rhéologique

La dégradation enzymatique des copolymères entraîne l'apparition de chaînes moléculaires de plus petites tailles et devrait entraîner par conséquent la diminution de la viscosité du milieu dans lequel ils sont en suspension.

Une suspension de copolymère de l'exemple 1 dans le mélange tampon (tris/acétate/albumine) est préparée dans les mêmes conditions opératoires que celles utilisées dans l'étude spectroscopique telle présentée ci-dessus. Puis, 4 ml de suspension sont incubés dans le cylindre du rhéomètre (RS100 *Haake*) maintenu à 37°C. La mesure de la viscosité (h) initiale est effectuée. Ensuite, 0,8 UE de chondroïtinases dissoutes dans 160 ml d'eau sont rajoutées à la suspension. Le témoin est une suspension de copolymère de l'exemple 1 préparée dans les conditions opératoires précédemment décrites, sans ajout d'enzyme et diluée dans 160 ml d'eau. L'évolution de la viscosité est suivie au cours du temps. L'essai est effectué deux fois pour chaque test.

La figure 2 est une représentation semi-logarithmique de l'évolution de la viscosité de la suspension du copolymère de l'exemple 1 en présence d'enzymes (ligne continue) ou en absence d'enzymes (ligne témoin en pointillé). La viscosité du témoin qui est de l'ordre de 17± 3 mPa.s, ne varie pas au cours du temps. Par contre, en présence d'enzymes, la viscosité chute progressivement de 17 mPa.s jusqu'à 3 mPa.s en 55 minutes puis devient quasi-stable. Cette chute importante de la viscosité s'explique par la dégradation du copolymère par les enzymes.

De plus, suite aux études spectroscopique et rhéologique ci-dessus menées dans les mêmes conditions opératoires, on peut remarquer, au bout de 55 minutes d'incubation en présence d'enzymes, une chute quasi totale de la viscosité alors qu'il n'y a seulement qu'une partie des disaccharides provenant de la dégradation de la CS qui est détectée en solution. Ainsi, la dégradation de quelques sites du copolymère par les enzymes suffit pour avoir un effondrement du réseau tridimensionnel du copolymère.

### Etude des comprimés à libération contrôlée

Les copolymères réticulés des exemples 1 à 6 sont tamisés puis mélangés avec de l'acide aminosalicylique (5ASA) et du stéarate de magnésium (rapport massique 79,5/20/0,5). Puis des comprimés de 250 mg et de dureté > 100 N sont préparés par compression directe.

Des essais de dissolution sont effectués sur les comprimés ainsi préparés, dans un appareillage à palette tournante (DISSOLUTEST) à 37°C sous une agitation de 50 tours/minutes. Les milieux de dissolution utilisés sont un mélange tampon de pH 1,2 et 7,5 correspondant respectivement aux milieux gastrique et intestinal artificiels (sans enzymes). Pour chaque formule et dans chaque milieu, l'essai est répété trois fois. A des temps déterminés, un échantillon du milieu de dissolution est prélevé et filtré. Le dosage du 5ASA est effectué par spectroscopie UV.

Le tableau 1 ci-dessous récapitule les temps (en heure) de libération de 50 % de la dose initiale du 5ASA (t_{50%}), obtenus en milieux gastrique et intestinal artificiels.

**Tableau 1**

| **Exemple** | t_{50%} (milieu gastrique) | t_{50%} (milieu intestinal) |
|---|---|---|
| 1 | 2,88 | 7,66 |
| 2 | 1,42 | 1,61 |
| 3 | 6,48 | 8,29 |
| 4 | 1,22 | 1,59 |
| 5 | 7,94 | 8,65 |
| 6 | 7,96 | 11,05 |

En milieu gastrique, les t_{50%} varient entre 1,2 et 8 heures permettant ainsi de moduler la libération du principe actif et cela en fonction de la nature des copolymères. Parmi ces copolymères, les copolymères des exemples 3, 5 et 6, ayant respectivement des t_{50%} de 6,5 ; 7,9 et 8 h, modèrent de façon importante la libération du principe actif.

En milieu intestinal, les t_{50%} varient entre 1,6 et 11 h permettant également de moduler la libération du principe actif et cela en fonction de la nature des copolymères. De plus, une modération importante de la libération du principe actif est obtenue avec les copolymères des exemples 1, 3, 5 et 6 . En effet les t_{50%} obtenus avec ces copolymères sont respectivement de 7,7 ; 8,3 ; 8,7 et 11 h.
Ainsi, les copolymères synthétisés permettent de réaliser des systèmes pharmaceutiques à libération contrôlée et cela en fonction des caractéristiques des copolymères réticulés. Plus particulièrement, ceux qui possèdent la propriété de modérer de façon importante la libération du principe actif et d'être dégradables par les chondroïtinases, semblent être des candidats intéressants pour réaliser des systèmes à libération au niveau du colon par action de la flore microbienne.

## Revendications

1. Copolymères réticulés à base de polymères polycarboxyliques non réticulés et d'un agent de réticulation comprenant au moins deux fonctions amine, les dits copolymères comprenant au moins un polysaccharide polycarboxylique et au moins un autre polymère polycarboxylique non réticulé qui n'est pas un polysaccharide polycarboxylique.

2. Copolymères selon la revendication 1, **caractérisés en ce que** le polysaccharide polycarboxylique est choisi parmi les glycosaminoglycanes, l'acide pectinique ou alginique.

3. Copolymères selon l'une des revendications 1 à 2, **caractérisés en ce que** le polysaccharide polycarboxylique est un glycosaminoglycane choisi parmi l'acide hyaluronique, la chondroïtine sulfate, l'héparine, le sulfate de dermatane, le sulfate d'héparane, le sulfate de kératane.

4. Copolymères selon l'une des revendications 1 à 3, **caractérisés en ce que** le polymère polycarboxylique non réticulé qui n'est pas un polysaccharide polycarboxylique est choisi parmi les polymères acryliques polycarboxyliques, le poly(acide glutamique), le poly(acide aspartique), le poly(acide maléique), le poly(acide malique) ou le poly(acide fumarique).

5. Copolymères selon l'une quelconque des revendications 1 à 4, **caractérisés en ce que** le polymère polycarboxylique non réticulé qui n'est pas un polysaccharide polycarboxylique, est un polymère acrylique polycarboxylique.

6. Copolymères selon la revendication 5, **caractérisés en ce que** le polymère acrylique polycarboxylique est le poly(acide acrylique) ou le poly(acide méthacrylique).

7. Copolymères selon l'une quelconque des revendications 1 à 6, dans lesquels l'agent de réticulation est choisi parmi les diamines, les acides aminés naturels ou synthétiques ou les polyamines.

8. Copolymères selon la revendication 7 dans lesquels l'acide aminé est choisi parmi la lysine, l'histidine ou l'ornithine.

9. Copolymères selon la revendication 7 dans lesquels la diamine est choisie parmi l'éthylènediamine, la butanediamine, l'hexanediamine, l'heptanediamine, l'octanediamine et la dodécanediamine.

10. Copolymères selon la revendication 7 dans lesquels la polyamine est choisie parmi le chitosane, la polyornithine ou la polylysine.

11. Copolymères selon l'une des revendications 1 à 10, **caractérisés en ce que** le polysaccharide polycarboxylique est dégradable par la flore du colon.

12. Copolymères selon la revendication 11, **caractérisés en ce que** le polysaccharide polycarboxylique est choisi parmi la chondroïtine sulfate, l'acide hyaluronique, l'acide pectinique ou l'héparine.

13. Copolymères selon l'une quelconque des revendications 1 à 8, 11 et 12, **caractérisés en ce que** le polysaccharide polycarboxylique est la chondroïtine sulfate, l'autre dit polymère polycarboxylique est le poly(acide acrylique) ou le poly(acide méthacrylique), et l'agent de réticulation est la lysine ou l'histidine.

14. Procédé de préparation de copolymères selon l'une quelconque des revendications 1 à 13, **caractérisé en ce que** l'on fait réagir, en milieu aqueux, les dits polymères polycarboxyliques non réticulés, en présence d'un activateur et dudit agent de réticulation.

15. Procédé selon la revendication 14, dans lequel l'activateur est choisi parmi les carbodiimides, les dérivés des quinolines et les anhydrides mixtes.

16. Composition pharmaceutique contenant au moins un principe actif et, à titre de support inerte ou d'excipient, au moins un copolymère selon l'une des revendications 1 à 10.

17. Composition pharmaceutique contenant au moins un principe actif et, à titre de support inerte ou d'excipient, au moins un copolymère selon l'une des revendications 11 à 13.

18. Utilisation d'une composition pharmaceutique selon l'une des revendications 16 à 17 pour une libération contrôlée.

19. Utilisation d'une composition pharmaceutique selon l'une des revendications 16 à 17 en tant que système pharmaceutique bioadhésif.

20. Utilisation d'une composition pharmaceutique selon la revendication 17 pour une libération spécifique du principe actif au niveau du colon.

21. Utilisation selon la revendication 20 pour véhiculer le principe actif destiné au traitement des maladies du colon.

22. Utilisation selon la revendication 20 pour véhiculer le principe actif qui est absorbé au niveau du colon.

23. Utilisation selon la revendication 20 pour véhiculer le principe actif qui est dégradé dans les parties hautes du tube digestif.

## Patentansprüche

1. Vernetzte Copolymere auf der Basis von nicht vernetzten Polycarboxylpolymeren und eines Vernetzungsmittels mit mindestens zwei, Aminfunktionen, wobei diese Copolymere mindestens ein Polycarboxylpolysaccharid und mindestens ein anderes nicht vernetztes Polycarboxylpolymer umfassen, das nicht ein Polycarboxylpolysaccharid ist.

2. Copolymere nach Anspruch 1, **dadurch gekennzeichnet, dass** das Polycarboxylpolysaccharid aus den Glucosaminoglykanen, pektiniger Säure oder Alginsäure ausgewählt ist.

3. Copolymere nach einem der Ansprüche 1 bis 2, **dadurch gekennzeichnet, dass** das Polycarboxylpolysaccharid ein Glucosaminoglykan ist, das aus Hyaluronsäure, Chondroitinsulfat, Heparin, Dermatansulfat, Heparansulfat und Keratansulfat ausgewählt ist.

4. Copolymere nach einem der Ansprüche 1 bis 3, **dadurch gekennzeichnet, dass** das nicht vernetzte Polycarboxylpolymer, das nicht ein Polycarboxylpolysaccharid ist, aus den Polycarboxylacrylpolymeren, Poly(glutaminsäure), Poly(asparginsäure), Poly(maleinsäure), Poly(äpfelsäure) oder Poly(fumarsäure) ausgewählt ist.

5. Copolymere nach einem der Ansprüche 1 bis 4, **dadurch gekennzeichnet, dass** das nicht vernetzte Polycarboxylpolymer, das nicht ein Polycarboxylpolysaccharid ist, ein Polycarboxylacrylpolymer ist.

6. Copolymere nach Anspruch 5, **dadurch gekennzeichnet, dass** das Polycarboxylacrylpolymer Poly(acrylsäure) oder Poly(methacrylsäure) ist.

7. Copolymere nach einem der Ansprüche 1 bis 6, bei denen das Vernetzungsmittel aus den Diaminen, den natürlichen oder synthetischen Aminosäuren oder den Polyaminen ausgewählt ist.

8. Copolymere nach Anspruch 7, bei denen die Aminosäure aus Lysin, Histidin oder Ornithin ausgewählt ist.

9. Copolymere nach Anspruch 7, bei denen das Diamin aus Ethylendiamin, Butandiamin, Hexandiamin, Heptandiamin, Octandiamin und Dodecandiamin ausgewählt ist.

10. Copolymere nach Anspruch 7, bei denen das Polyamin aus Chitosan, Polyornithin oder Polylysin ausgewählt ist.

11. Copolymere nach einem der Ansprüche 1 bis 10, **dadurch gekennzeichnet, dass** das Polycarboxylpolysaccharid durch die Darmflora abbaubar ist.

12. Copolymere nach Anspruch 11, **dadurch gekennzeichnet, dass** das Polycarboxylpolysaccharid aus Chondroitinsulfat, Hyaluronsäure, pektiniger Säure oder Heparin ausgewählt ist.

13. Copolymere nach einem der Ansprüche 1 bis 8, 11 und 12, **dadurch gekennzeichnet, dass** das Polycarboxylpolysaccharid Chondroitinsulfat ist, das andere Polycarboxylpolymer Poly(acrylsäure) oder Poly(methacrylsäure) ist und das Vernetzungsmittel Lysin oder Histidin ist.

14. Verfahren zur Herstellung von Copolymeren nach einem der Ansprüche 1 bis 13, **dadurch gekennzeichnet, dass** man die nicht vernetzten Polycarboxylpolymere in wässrigem Medium in Gegenwart eines Aktivators und des Vernetzungsmittels reagieren lässt.

15. Verfahren nach Anspruch 14, bei dem der Aktivator aus den Carbodiimiden, den Derivaten der Chinoline und den gemischten Anhydriden ausgewählt ist.

16. Pharmazeutische Zusammensetzung, die mindestens einen Wirkstoff und als neutralen Träger oder Grundmasse mindestens ein Copolymer nach einem der Ansprüche 1 bis 10 enthält.

17. Pharmazeutische Zusammensetzung, die mindestens einen Wirkstoff und als neutralen Träger oder Grundmasse mindestens ein Copolymer nach einem der Ansprüche 11 bis 13 enthält.

18. Verwendung einer pharmazeutischen Zusammensetzung nach einem der Ansprüche 16 bis 17 für eine gesteuerte Freisetzung.

19. Verwendung einer pharmazeutischen Zusammensetzung nach einem der Ansprüche 16 bis 17 als bioadhäsives pharmazeutisches System.

20. Verwendung einer pharmazeutischen Zusammensetzung nach Anspruch 17 für eine spezifische Freisetzung des Wirkstoffs im Dickdarmbereich.

21. Verwendung nach Anspruch 20 zur Beförderung des für die Behandlung von Dickdarmerkrankungen bestimmten Wirkstoffs.

22. Verwendung nach Anspruch 20 zur Beförderung des Wirkstoffs, der im Dickdarmbereich absorbiert wird.

23. Verwendung nach Anspruch 20 zur Beförderung des Wirkstoffs, der in den oberen Teilen des Verdauungstrakts abgebaut wird.

## Claims

1. Cross-linked copolymers based on non cross-linked polycarboxylic polymers and a cross-linking agent comprising at least two amine functions, said copolymers comprising at least one polycarboxylic polysaccharide and at least one other non cross-linked polycarboxylic polymer which is not a polycarboxylic polysaccharide.

2. Copolymers according to claim 1, **characterized in that** the polycarboxylic polysaccharide is chosen from glycosaminoglycans, pectinic or alginic acid.

3. Copolymers according to one of claims 1 to 2, **characterized in that** the polycarboxylic polysaccharide is a glycosaminoglycan chosen from hyaluronic acid, chondroitin sulphate, heparin, dermatan sulphate, heparan sulphate, keratan sulphate.

4. Copolymers according to one of claims 1 to 3, **characterized in that** the non cross-linked polycarboxylic polymer which is not a polycarboxylic polysaccharide is chosen from polycarboxylic acrylic polymers, poly(glutamic acid), poly(aspartic acid), poly(maleic acid), poly(malic acid) or poly(fumaric acid).

5. Copolymers according to any one of claims 1 to 4, **characterized in that** the non cross-linked polycarboxylic polymer which is not a polycarboxylic polysaccharide is a polycarboxylic acrylic polymer.

6. Copolymers according to claim 5, **characterized in that** the polycarboxylic acrylic polymer is poly(acrylic acid) or poly(methacrylic acid).

7. Copolymers according to any one of claims 1 to 6, in which the cross-linking agent is chosen from diamines, natural or synthetic amino acids or polyamines.

8. Copolymers according to claim 7 in which the amino acid is chosen from lysine, histidine or ornithine.

9. Copolymers according to claim 7 in which the diamine is chosen from ethylenediamine, butanediamine, hexanediamine, heptanediamine, octanediamine and dodecanediamine.

10. Copolymers according to claim 7 in which the polyamine is chosen from chitosan, polyornithine or polylysine.

11. Copolymers according to one of claims 1 to 10, **characterized in that** the polycarboxylic polysaccharide can be degraded by the flora of the colon.

12. Copolymers according to claim 11, **characterized in that** the polycarboxylic polysaccharide is chosen from chondroitin sulphate, hyaluronic acid, pectinic acid or heparin.

13. Copolymers according to any one of claims 1 to 8, 11 and 12, **characterized in that** the polycarboxylic polysaccharide is chondroitin sulphate, the other said polycarboxylic polymer is poly(acrylic acid) or poly(methacrylic acid) and the cross-linking agent is lysine or histidine.

14. Process for the preparation of copolymers according to any one of claims 1 to 13, **characterized in that** said non cross-linked polycarboxylic polymers are reacted in an aqueous medium, in the presence of an activator and of said cross-linking agent.

15. Process according to claim 14, in which the activator is chosen from carbodiimides, quinoline derivatives and mixed anhydrides.

16. Pharmaceutical composition containing at least one active ingredient and, as an inert support or excipient, at least one copolymer according to one of claims 1 to 10.

17. Pharmaceutical composition containing at least one active ingredient and, as an inert support or excipient, at least one copolymer according to one of claims 11 to 13.

18. Use of a pharmaceutical composition according to one of claims 16 to 17 for sustained release.

19. Use of a pharmaceutical composition according to one of claims 16 to 17 as a bioadhesive pharmaceutical system.

20. Use of a pharmaceutical composition according to claim 17 for a specific release of the active ingredient at the level of the colon.

21. Use according to claim 20 to convey the active ingredient intended for the treatment of diseases of the colon.

22. Use according to claim 20 to convey the active ingredient which is absorbed at the level of the colon.

23. Use according to claim 20 to convey the active ingredient which is degraded in the upper parts of the digestive tube.
